# EUROPEAN PATENT APPLICATION

(11) **EP 3 173 109 A1**
(43) Date of publication of application: **31.05.2017**
(21) Application number: 15197077.9
(22) Date of filing: 30.11.2015
(51) Int. Cl.: A61M 1/10

(54) **METHOD FOR CONTROLLING A CENTRIFUGAL HEART ASSIST POMP IMPELLER POSITION**

(71) Applicant: Fundacja Rozwoju Kardiochirurgii Im. Prof. Zbigniewa Religi, 41-800 Zabrze (PL)
(72) Inventor: KUSTOSZ, Roman, 41-800 Zabrze (PL); DARLAK, Maciej, 41-710 Ruda l ka (PL); ALTYNTSEV, Ievgenii, 40-024 Sumy (UA); JUNAK, Jacek, 41-902 Bytom (PL); GAWLIKOWSKI, Maciej, 41-800 Zabrze (PL); CZAK, Miroslaw, 40-748 Katowice (PL)
(74) Representative: Malcherek, Piotr

(57) **Abstract**

A method of controlling the position of an impeller of a centrifugal ventricular assist pump, in which the impeller rotates around an axis inside the pump without mechanical contact with the pump housing, characterised in that the axial position of the impeller is monitored through the analysis of amplitudes of signals of the reverse electromotive force induced in non-powered phases of two three-phase motors of the pump arranged opposite each other on the axis of rotation of the impeller on either side thereof in the upper and lower part of the pump housing, and the position of the impeller along its axis of rotation is adjusted by changing the control parameters of one motor with respect to the other, which results in producing a difference in electromagnetic forces generated by a system of the two aforementioned motors.

## Description

### THE INVENTION

The invention relates to a method of controlling the position of an impeller of a centrifugal ventricular assist pump, said method provided for controlling the position of the impeller in ventricular assist pumps with the impeller suspended within the blood pump chamber by means of magnetic and hydrodynamic bearings, without mechanical contact with the pump housing.

### STATE OF THE ART

In the state of the art, for example in EP2405141, there are known impellers of ventricular assist pumps suspended within the pump housing with the use of a hybrid bearing comprising a hydrodynamic and a magnetic bearing. The pump impeller is driven by symmetrical, three-phase, brushless and sensorless motors mounted in the upper and lower part of the pump housing, said motors cooperating with the impeller mounted therebetween. Said impeller is equipped with permanent magnets arranged in a ring shape in the upper and lower impeller shrouds. Each of said motors comprises a magnetic core physically and magnetically connecting a set of nine coils electrically connected into three electrical phases. Each electrical limb comprises three coils geometrically shifted by 120 degrees. The use of a hybrid bearing and the impeller driven by motors coupled with magnets in the impeller enables balancing the forces affecting the impeller in such a way that the position of the impeller in the pump housing is controlled while avoiding contact between the impeller and the housing, smooth actuation therefore being ensured.

### THE ESSENCE OF THE INVENTION

The invention relates to a method of controlling the position of an impeller of a centrifugal ventricular assist pump, wherein said impeller rotates around an axis within the pump without mechanical contact with the pump housing. The essence of the invention consists in that the axial position of the impeller is monitored through the analysis of amplitudes of signals of the reverse electromotive force, said signals being induced in non-powered phases of two three-phase motors of the pump, said motors being arranged opposite each other on the axis of rotation of the impeller on either side thereof in the upper and lower part of the pump housing, and the position of the impeller along its axis of rotation is adjusted by changing the control parameters of one motor with respect to the other. This results in producing a difference in electromagnetic forces generated by a system of the two aforementioned motors, and thereby a change in the axial position of the impeller is achieved.

Preferably the position of the impeller is adjusted by changing the phase shift between the synchronically generated impulses of a cyclical current wave supplied on the coils of the motors.

It is also possible to adjust the position of the impeller by changing the energy levels supplied to the electromagnetic coils of one motor with respect to the other, which results in a change in magnetic field strength produced by the motors.

### ADVANTAGES OF THE INVENTION

The method of controlling the position of an impeller of a ventricular assist pump according to the invention enables to move the impeller in the pump along the axis of rotation without the use of additional construction elements of the pump such as coils, magnets or cables. It is particularly advantageous that the impeller can be moved without changing the rotational speed thereof, which results in maintaining constant pump efficiency.

The fact that the impeller moves inside the pump enables blood to flow over the entire impeller, thus eliminating the risk of thrombus formation above and below the impeller in the area of the hydraulic bearings which are particularly prone to thrombus formation due to a confined space between the impeller and the pump housing and slow flow of blood in these areas.

Moreover, the application of the method according to the invention for monitoring the axial position of the impeller eliminates the need to use additional measurement systems for monitoring the position of the impeller, which results in decreasing the overall weight of the pump.

### DESCRIPTION OF THE EMBODIMENT DRAWING

The method is presented in more detail in the embodiment and the enclosed drawing, where:

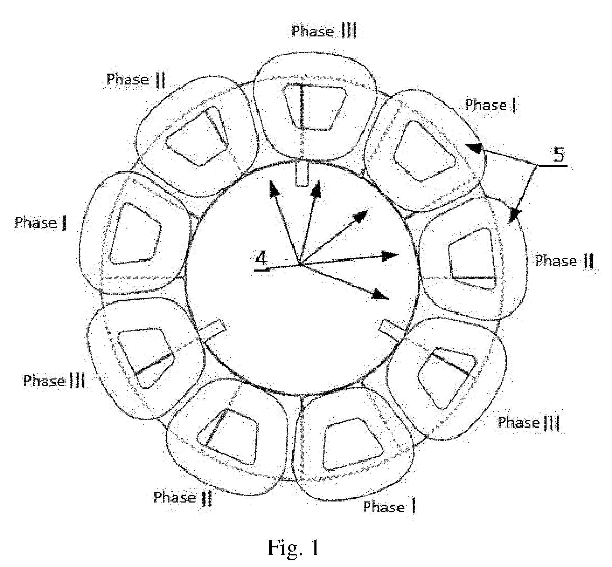

Fig. 1 is a schematic view of the power system of the motors in connection with the impeller,

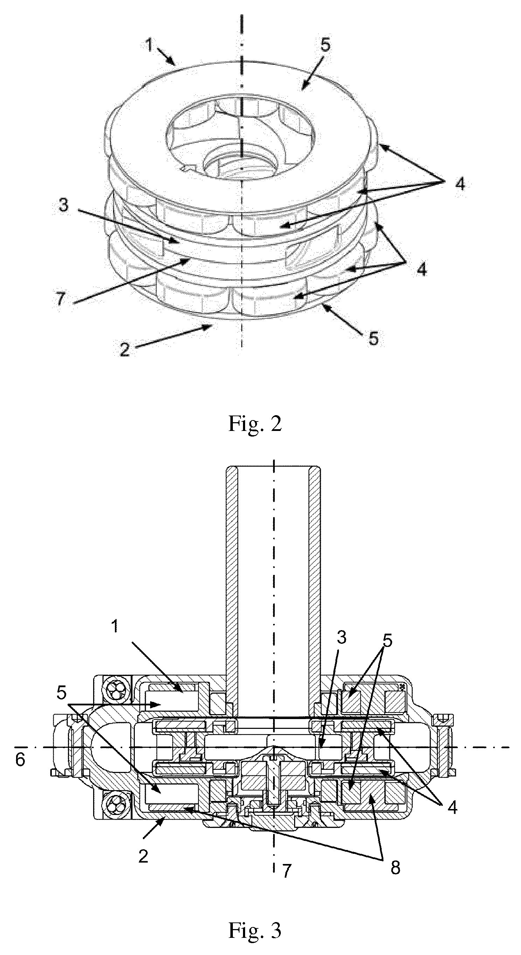

Fig. 2 is an isometric projection of the centrifugal blood pump,
Fig. 3 is a cross-section of the centrifugal blood pump,

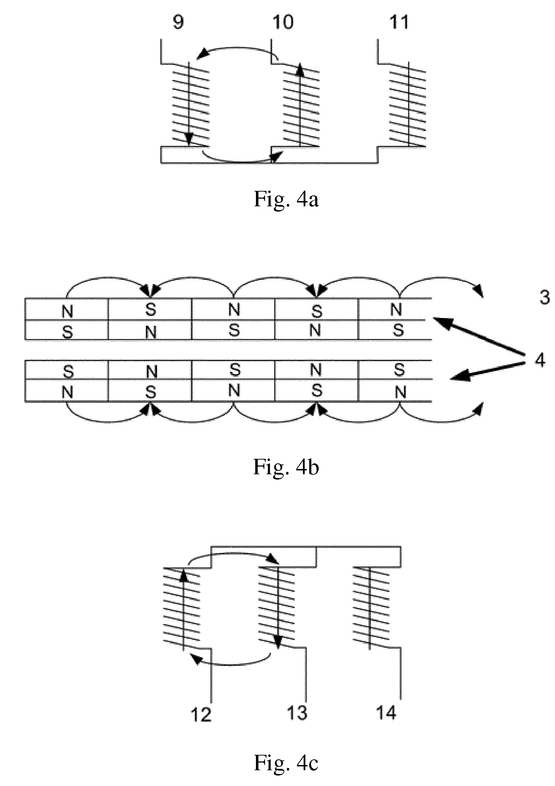

Fig. 4a - Fig. 4c are a diagram of magnetic fields distribution with the supply of Phases I and II.

### EMBODIMENT

The method of controlling the position of an impeller was presented in an embodiment, in which said method was applied in a centrifugal ventricular assist pump.

The ventricular assist pump comprises a housing inside which there is an impeller comprising two shrouds between which there are flow channels for blood. The pump housing is built of two parts constituting an upper and a lower pump housing. In both said housing parts there are identical three-phase, brushless motors. In both said housing parts it is also possible to use identical three-phase, brushless, sensorless motors. In both the upper shroud and the lower shroud of the impeller there are twelve permanent magnets arranged in a ring shape forming six pairs of magnetic fields shifted in the phase by 30 degrees. The magnets on the impeller shrouds are mutually adjacent, and at the same time they lie in the planes perpendicular to the axis of rotation of the impeller and are arranged at the same distance from the geometric centre of the impeller. The so constructed impeller is a common rotor for the motors located in the upper and lower pump housing. In more detail the magnets of the first shroud of the impeller are the rotor of the motor located in the upper pump housing, and the magnets of the second shroud of the impeller are the rotor of the motor located in the lower pump housing.

The stator of each of the motors mounted in the pump housing comprises a magnetic core physically and magnetically connecting a set of nine coils electrically connected into three electrical phases (I, II, III). In each of the motors the distance between the magnetic core and the coils of the stator and magnets mounted on the shroud of the impeller constituting a rotor should be the same. Most preferably the distance should be 0.85 mm. Each limb of the circuit comprising three coils producing appropriate phases (I, II, III) is geometrically shifted by an angle of 120 degrees. The phases of the motor are mutually shifted by 40 degrees, thus electrically forming a star system. In operation the phases are supplied in pairs (I-II, II-III, III-I) producing a magnetic field phase shifted by 40 degrees between the adjacent coils. While the pump is at work the spinning magnetic field induced in the coils is linked to the magnetic field produced by the twelve permanent magnets ring arranged in the shrouds of the impeller.

In the pump, in which the method of controlling the position of the impeller was applied in order to enhance hemocompatibility, a mechanical bearing was not used. The suspension of the impeller was constructed with the functional form of two contactless bearings: a magnetic bearing and a hydrodynamic bearing forming a hybrid bearing. The magnetic bearing is built of ring-shaped neodymium permanent magnets mounted in the impeller and in the pump housing. The aim of said bearing is to stabilize the impeller in the radial direction. The elements of the hydrodynamic bearing are located in the upper and in the lower shroud of the impeller. The aim of the hydrodynamic bearing is to prevent contact of the impeller with the pump housing in the axial direction as well as to maintain a safe gap between the impeller and the pump housing. The width of the gap between the impeller and the pump housing should not be smaller than 50 µm, which is several times larger than even the largest morphotic elements of blood. The size of said gap changes depending on the position of the impeller of the centrifugal ventricular assist pump.

In this centrifugal ventricular assist pump the axial position of the impeller is monitored through the analysis of amplitudes of signals of the reverse electromotive force, said signals being induced in non-powered phases of two three-phase motors of the pump. The position of the impeller along its axis of rotation is adjusted by changing the control parameters of one motor with respect to the other, which results in producing a difference in electromagnetic forces generated by a system of the two aforesaid motors.

In particular by changing the phase shift between the synchronically generated impulses of the cyclical current wave supplied on the coils of the motors, the position of the impeller along its axis of rotation is changed and adjusted.

It is also possible to adjust the position of the impeller by changing the level of energy supplied to the electromagnetic coils of one motor with respect to the other. This results in a change in strength of the magnetic field produced by the motors.

Controlling the position of the impeller inside the pump does not affect the rotational speed of the impeller or the pump efficiency.

### REFERENCE LIST

1. Upper motor
2. Lower motor
3. Impeller
4. Drive magnets in the impeller
5. Coils
6. The plane of the geometric centre of the impeller position in the pump housing
7. Axis of rotation of the impeller
8. Magnetic core
9. PHASE I of the upper motor
10. PHASE II of the upper motor
11. PHASE III of the upper motor
12. PHASE I of the lower motor
13. PHASE II of the lower motor
14. PHASE III of the lower motor

## Claims

1. A method of controlling the position of an impeller of a centrifugal ventricular assist pump, in which the impeller rotates around an axis inside the pump without mechanical contact with the pump housing, **characterised in that** the axial position of the impeller is monitored through the analysis of amplitudes of signals of the reverse electromotive force induced in non-powered phases of two three-phase motors of the pump arranged opposite each other on the axis of rotation of the impeller on either side thereof in the upper and lower part of the pump housing, and the position of the impeller along its axis of rotation is adjusted by changing the control parameters of one motor with respect to the other, which results in producing a difference in electromagnetic forces generated by a system of the two aforementioned motors.

2. The method according to claim 1, **characterised in that** the position of the impeller is adjusted by changing a phase shift between synchronically generated impulses of a cyclical current wave supplied on the coils of the motors.

3. The method according to claim 1, **characterised in that** the position of the impeller is adjusted by changing the levels of energy supplied on the electromagnetic coils of one motor with respect to the other resulting in the change in strength of the magnetic field produced by the motors.
